# EUROPÄISCHE PATENTSCHRIFT

(11) **EP 2 200 752 B1**
(45) Veröffentlichungstag und Bekanntmachung des Hinweises auf die Patenterteilung: **31.10.2012**
(21) Anmeldenummer: 09716396.8
(22) Anmeldetag: 04.03.2009
(51) Int. Cl.: B05B 11/00, A61L 2/238

(54) **DOSIERUNGSVORRICHTUNG**
METERING DEVICE
DISPOSITIF DE DOSAGE

(30) Priorität: 04.03.2008 DE 102008012468; 12.06.2008 DE 102008027987
(43) Veröffentlichungstag der Anmeldung: 30.06.2010
(73) Patentinhaber: F. Holzer GmbH, 66386 St. Ingbert (DE); KIST-Europe Forschungsgesellschaft mbH, 66123 Saarbrücken (DE)
(72) Erfinder: LEE, Hyeck-Hee, 66386 St. Ingbert (DE); STEINFELD, Ute, 66386 St. Ingbert (DE); KIM, Chang-Ho, Seoul (KR); KIM, Jungtae, 66121 Saarbrücken (DE); KRAUSE, Holger, 66540 Neunkirchen (DE)
(74) Vertreter: Pfenning, Meinig & Partner GbR
(86) Internationale Anmeldenummer: PCT/EP2009/001528
(87) Internationale Veröffentlichungsnummer: WO 2009/109370

(56) Entgegenhaltungen:
- EP-A- 0 473 892
- EP-A- 0 765 690
- EP-A- 1 327 478
- EP-A- 1 380 352
- DE-A1- 10 330 040
- DE-A1-102006 024 563
- US-A- 6 082 592

## Beschreibung

Vorliegende Erfindung betrifft eine Dosierungsvorrichtung zur dosierten Abgabe von Flüssigpräparaten, insbesondere zur Dosierung von medizinischen, pharmazeutischen und kosmetischen Flüssigpräparaten, wobei auf die Verwendung von Konservierungsmitteln verzichtet werden kann. Weitere Verwendungen existieren im Lebensmittelbereich z.B. für Nahrungsergänzungsmittel, Gewürze etc.

Eine Dosierungsvorrichtung zur Abgabe von keimfreiem Fluid ist beispielsweise aus der EP 0 473 892 A2 bekannt. Die hierin geschilderte Dosierungsvorrichtung ist allerdings aus sehr vielen Einzelteilen aufgebaut, was sowohl die Herstellung unnötig kompliziert und verteuert sowie die geschilderte Dosierungsvorrichtung fehler- und störanfällig werden lässt.

Weiterhin ist bei dieser vorbekannten Dosiervorrichtung nicht in allen Fällen eine hinreichend genaue Dosierung des zu dosierenden Präparats möglich.

Aus der DE 103 30 040 A1 ist ein Spender für pastöse Massen, der durch Fingerdruck auf einen Dosierkopf betätigbar ist, bekannt. Ein Ausgabeventil verschließt im Ruhezustand luftdichten Massebereich, was durch die fehlende Verspannung eines Steuerelementes erzielbar ist.

Die EP 1 380 352 A1 betrifft ebenso eine Saug-Druck-Pumpe zum Ausspritzen eines Produktes aus einem Behälter, die ein Gehäuse, in dem ein Kolben über einen ersten Gehäuseabschnitt abgedichtet verschiebbar ist, aufweist. Eine eine Kolbenbohrung festsetzende hohle Kolbenstange ist in einer Verschlusshaube von Gehäuse und Behältnis verschiebbar und mit einem Betätigungskopf versehen, durch dessen Betätigung der Kolben gegen eine Rückstellfederkraft zu einem Tauchrohr-Anschlussstutzen am Gehäuse gedrückt wird und dabei in einem Druckraum einen Druck aufbaut, der ein erstes Ventil im Anschlussstutzen abschließt.

Zudem betrifft die DE 10 2006 024 563 A1 ein Verfahren zur Herstellung einer befüllten Dosierpumpenanordnung mit folgenden Schritten: Einfüllen eines fließfähigen Produktes in einen einseitig offenen Folienbeutel oder dergleichen, Verschweißen des Folienbeutels durch einen Deckel und/oder eine ggf. erste vormontierte manuelle betätigbare Pumpe, die im unbelasteten Zustand durch wenigstens ein Rückschlagventil eine Fluidverbindung zwischen einer mit der Umgebung in Verbindung stehenden Auslassöffnung der Pumpe und dem Innenraum des Folienbeutels absperrt, und zumindest näherungsweise vollständiges Entfernen der in dem Folienbeutel befindlichen Gase.

Weiterhin betrifft die EP 1 327 478 A1 einen Betätigungskopf einer Saug-Druck-Pumpe zum Ausspritzen eines Produktes aus einem Behältnis, der einen zu einer Ausspritzöffnung führenden Auslasskanal, in dem ein durch eine Feder belastetes Verschlussstück angeordnet ist.

Die EP 0 765 690 A1 betrifft ein Dispensionssystem, mit einer Dispensionspumpe, die auf dem obersten Bereich eines Behältnisses angeordnet ist.

Die US 6,082,592 betrifft einen Zerstäuber mit einem Behältnis für Reinwasser. Auf dem Behältnis ist eine Pumpe montiert, mit der Wasser aus dem Behältnis zerstäubt werden kann.

Ausgehend hiervon ist es Aufgabe der vorliegenden Erfindung, eine Dosierungsvorrichtung zur dosierten Abgabe von Flüssigpräparaten bereitzustellen, die einen möglichst simplen Aufbau aufweist und somit die aus dem Stand der Technik bekannten Nachteile umgeht.

Diese Aufgabe wird mit den Merkmalen des Patentanspruchs 1 gelöst. Die abhängigen Ansprüche stellen dabei vorteilhafte Weiterbildungen dar.

Erfindungsgemäß wird somit vorgeschlagen, die aus dem Stand der Technik bekannte Dosiervorrichtung so weiterzubilden, dass im Bereich des Endes des Auslasskanals ein federbetriebenes Auslassventil angeordnet ist, wobei dessen Feder in einer separaten, mit dem Auslasskanal in Verbindung stehenden Aussparung geführt ist.

Durch die Anordnung des Auslassventils im Bereich des

Endes des Auslasskanals wird eine optimale Dosierung von zu dosierenden Präparaten unter allen Betriebsbedingungen erreicht. Insbesondere ist bei dieser Lösung vorteilhaft, dass bei längerem Stehen der Dosiervorrichtung es zu keiner Eintrocknung des im Auslasskanal zur Düse befindlichen Flüssigpräparates kommt, ebenso wird eine Kontamination, beispielsweise durch Bakterien, der in dem Auslasskanal befindlichen Flüssigkeit effektiv verhindert. Das Auslassventil gemäß der Erfindung ist dabei so aufgebaut, dass eine Feder, d.h. eine Ventilfeder in einer Aussparung, vorteilhafterweise einer Nut, geführt ist, die mit dem Auslasskanal in Verbindung steht und zum Betrieb des Auslassventils, d.h. des Ventilkolbens, dient. Wenn nun die Dosiervorrichtung der Erfindung betätigt wird, übt die aus dem Innenraum der Dosiervorrichtung beförderte Flüssigkeit einen Druck gegen die Ventilfeder und den Ventilkolben aus, so dass sich der Kolben in Richtung Feder bewegt und die Austrittsöffnung freigibt, wodurch die Flüssigkeit austreten kann. Dadurch, dass nun die Ventilfeder in einer separaten Aussparung geführt ist, wird zudem gewährleistet, dass das Flüssigpräparat, das dosiert werden soll, mit der Feder nicht in Kontakt kommt.

Bei der erfindungsgemäßen Dosiervorrichtung kann dabei die Düse und der Auslasskanal entweder seitlich aus dem Hohlkörper, der bevorzugt als Hohlzylinder ausgebildet ist, herausgeführt werden oder aber die Düse wird über die Kappe nach außen geführt.

Eine weitere vorteilhafte Ausführungsform der Erfindung schlägt vor, dass der Auslasskanal und/oder der Ventilkörper ein bakterizid wirkendes Mittel aufweisen. Dies kann dergestalt erfolgen, dass die betreffenden Teile entweder direkt aus dem bakterizid wirkenden Mittel gebildet sind, wie beispielsweise Silber, oder dass die entsprechenden Bereiche eine bakterizid wirkende Beschichtung aufweisen. Ebenso ist die Möglichkeit gegeben, dass bakterizid wirkende Einbauten vorhanden sind, dies kann beispielsweise in Form einer Feder oder Spirale im Bereich der Düsenöffnung erfolgen, die eine bakterizide Wirkung aufweist.

Als bakterizid wirkende Mittel kommen insbesondere Silber oder Silbersalze, wie z.B. Silberchlorid, in Frage.

Die erfindungsgemäße Dosiervorrichtung zeichnet sich bevorzugt weiter dadurch aus, dass sie einen erheblich vereinfachten Aufbau aufweist. Dadurch, dass die Kappe mit dem Hohlkörper, der bevorzugt zylindrisch ist, einteilig ausgebildet sein kann, ist die Möglichkeit gegeben, den Betätigungskörper aus einer deutlich reduzierten Anzahl an Einzelteilen herzustellen und somit die Produktionskosten zu senken. Insbesondere ist es dabei bevorzugt, wenn nicht nur die Kappe mit dem Hohlzylinder, sondern auch noch der Pumpenkolben einstückig, d.h. monolithisch, ausgebildet ist. Beispielsweise lassen sich die zuvor genannten Einzelteile kostengünstig im Spritzgussverfahren aus quasi beliebigen Kunststoffen herstellen. Ein weiterer Vorteil ist darin zu sehen, dass die Verpackung bei gleich bleibendem Inhalt und gleicher Funktionalität wesentlich kleiner ausfällt, was unter ökonomischen wie ökologischen Aspekten gleichermaßen als vorteilhaft anzusehen ist.

Der Auslasskanal mit der Düse kann auch als integraler Bestandteil der Kappe ausgebildet sein, wobei in diesem Fall die Verbindung zur Pumpkammer bevorzugt durch eine im Pumpkolben verlaufende Kanal ausgebildet ist. Alternativ hierzu ist ebenso eine Konstruktion möglich, bei der der Auslasskanal mit der Düse als separates Bauteil vorhanden ist.

In einer vorteilhaften Ausführungsform ist die Pumpkammer über eine Einlassöffnung mit dem Vorratsbehältnis verbunden. Um ein effektives Nachströmen der zu dosierenden Flüssigkeit zu gewährleisten sowie ein Rückströmen der in der Pumpkammer befindlichen Flüssigkeit während des Dosiervorgangs zu verhindern, weist diese Einlassöffnung ein Einlassventil auf. Dieses Einlassventil kann beispielsweise als Kugelventil ausgebildet sein, und insbesondere aus bakterizid wirkenden Stoffen oder aus Materialien, die mit bakterizid wirkenden Stoffen beschichtet sind, wie beispielsweise Silber oder Silberchlorid gebildet sein. Besonders bevorzugt ist hierbei eine mit Silber beschichtete Edelstahlkugel als Einlassventil.

Um nach dem Dosiervorgang eine Regeneration des Betätigungskörpers sowie ein Nachfüllen von Flüssigkeit aus dem Vorratsbehältnis in die Pumpkammer zu gewährleisten, ist erfindungsgemäß zwischen der Pumpkammer und der Kappe eine mechanische Rückstellvorrichtung angeordnet. Diese ist ein Faltenbalg oder ein Faltenbalg mit Rückstellfeder.

Bei der erfindungsgemäßen Dosiervorrichtung ist es weiterhin günstig, wenn das Vorratsbehältnis an seiner Innenseite eine reibungsverminderte Ausstattung besitzt. Vorzugsweise ist die Innenseite mit einer Polyethylenschicht ausgestattet. Für diesen Fall kann das Vorratsbehältnis in einem Zweikomponenten-Spritzgussverfahren hergestellt werden. Neben der Innenseite des Vorratsbehältnisses kann auch gleichzeitig und/oder unabhängig hiervon der Kolben eine Gleitschicht aufweisen. Die in diesem Abschnitt beschriebene Beschichtung mit Polyethylen vermindert nicht nur die Reibung, sondern sie bietet auch den Vorteil, gegebenenfalls für medizinische Verwendungszwecke zugelassen zu sein.

Weiter bevorzugt ist, wenn die Dosiervorrichtung luftausgleichsfrei arbeitet, d.h. während ihrer Betätigung erfolgt kein Druckausgleich im Vorratsbehälter durch einströmende Luft.

Das Vorratsbehältnis kann auch einen Kolben, z.B. einen Schleppkolben, aufweisen. Der Boden des Vorratsbehältnisses kann mit einer Filtermatrix, z.B. einem Aktivkohlefilter oder einer Nylon- oder Polyvinylidenfluorid-Membran (PVDF), ausgerüstet sein, die sicherstellt, dass z.B. Bakterien und Sporen nicht mehr der einströmenden Luft passieren können.

Die vorliegende Erfindung wird anhand der nachfolgenden Figuren näher erläutert, ohne auf die in den Figuren dargestellten speziellen Ausführungen beschränkt zu sein.

Dabei zeigen
- Figur 1: in vergrößerter Darstellung den Betätigungskörper der erfindungsgemäßen Dosiervorrichtung mit einer ersten Ausführungsform des Auslassventils.
- Figur 2: zeigt ebenfalls wiederum in vergrößerter Darstellung den Betätigungskörper der erfindungsgemäßen Dosiervorrichtung hier mit einem schräg nach außen gerichteten Auslasskanal.
- Figur 3: zeigt ebenfalls in vergrößerter Darstellung den Betätigungskörper einer erfindungsgemäßen Dosiervorrichtung, wobei hier die Auslassdüse über die Kappe nach außen geführt ist.
- Figur 4: zeigt nun in der Gesamtansicht in der Figur 4a eine Dosiervorrichtung bestehend aus einem Betätigungskörper und einer Schleppkolbenflasche, wobei die Figur 4a den leeren Zustand und die Figur 4b den gefüllten Zustand der Schleppkolbenflasche zeigt.
- Figur 5: zeigt in Abwandlung der Figur 4a und b in den Figuren 5a und 5b eine Dosiervorrichtung mit einer Schleppkolbenflasche mit einer zusätzlichen Druckfeder unter dem Schleppkolben dar, wobei die Figur 5a den gefüllten Zustand und die Figur 5b den leeren Zustand zeigt.
- Figur 6: zeigt eine weitere Ausführungsform der erfindungsgemäßen Dosiervorrichtung, wobei hier das Vorratsbehältnis mit einem Faltenbalg ausgestattet ist. Die Figur 6a zeigt dabei den vollen Zustand der Dosiervorrichtung und Figur 6b die entleerte Variante.
- Figur 7: zeigt eine weitere Ausführungsform, wobei hier in Abwandlung zur Figur 6 unter dem Schleppkolben eine zusätzliche Druckfeder vorgesehen ist. Die Figur 7a zeigt den vollen Zustand des Vorratsbehältnisses und die Figur 7b dieselbe Dosiervorrichtung im entleerten Zustand.
- Figur 8: zeigt ausschnittsweise in vergrößerter Darstellung die Ausbildung eines beschichteten Schleppkolbens.

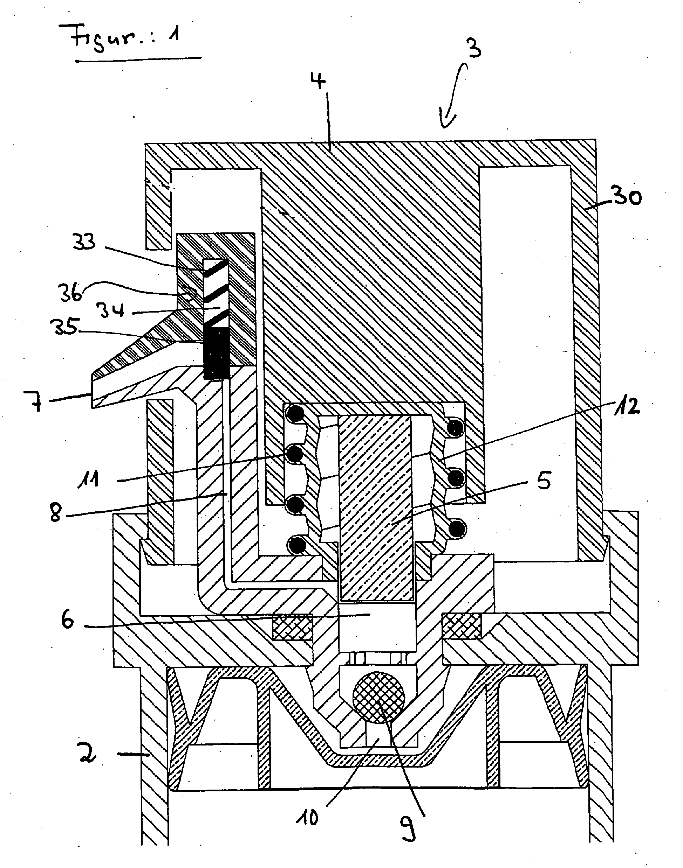

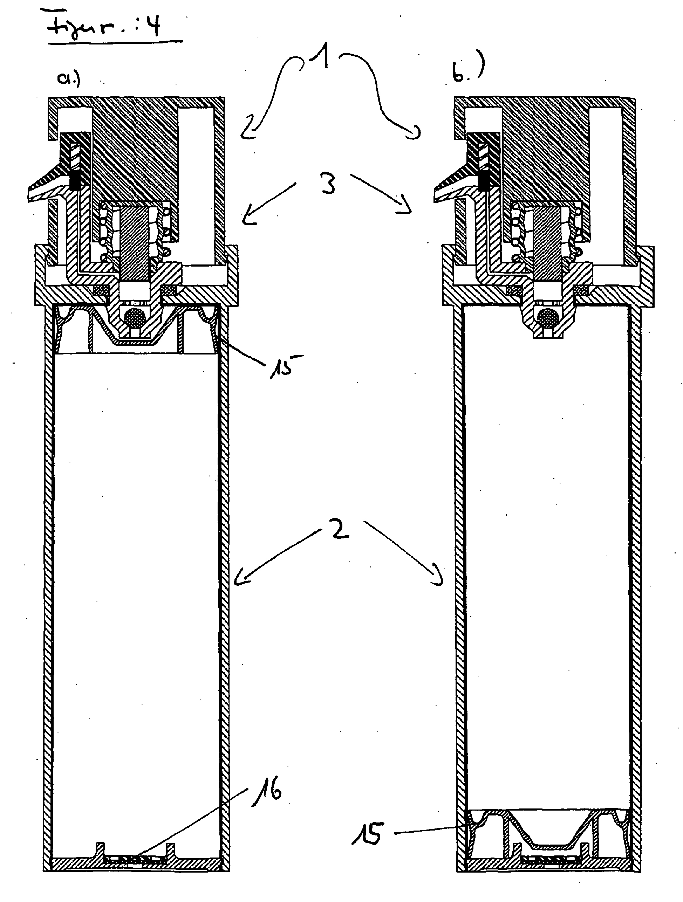

Figur 1 zeigt nun in vergrößerter Darstellung den Betätigungskörper 3 der erfindungsgemäßen Dosiervorrichtung 1. In Figur 1 ist das Vorratsbehältnis 2 in der Figur nur angedeutet. In Bezug zur Ausbildung auf das Vorratsbehältnis wird auf die Figuren 4 bis 7 verwiesen.

Der Betätigungskörper 3 der Ausführungsform nach Figur 1 besteht dabei aus einer Kappe 4 sowie aus einem Hohlkörper in zylindrischer Form 30 und einen einseitig mit der Kappe 4 und dem zylindrischen Hohlkörper 30 verbundenen Pumpkolben 5, der formschlüssig in die Pumpkammer 6 einbringbar ist. Wesentlich ist nun, dass im Bereich des Endes des Auslasskanals 8 ein Auslassventil 36, das aus einer Druckfeder 33 und einem Ventilkolben 35 besteht, angeordnet ist. Die Druckfeder 33 ist dabei in einer Aussparung 34, die mit dem Auslasskanal 8 in Verbindung steht, geführt. Durch Betätigen der Kappe 4 durch Aufdrücken durch den Anwender bewegt sich nun der Pumpkolben 5 in die Pumpkammer 6 und verdrängt dadurch die in der Pumpkammer 6 befindliche Flüssigkeit durch den Auslasskanal 8 zur Düse 7 hin. Durch den dabei entstehenden Überdruck wird das Auslassventil 36 gegen die Spannkraft der das Ventil 36 in der geschlossenen Position haltenden Feder 33 geöffnet, so dass ein Austritt der Flüssigkeit ermöglicht wird. Das Hubvolumen des Kolbens 5 bestimmt dabei die abgegebene dosierte Menge. Nach Beendigung des Dosiervorgangs wird durch die Rückstellfeder 11, die in einen Faltenbalg 12 eingearbeitet ist, die Kappe 4 mit verbundenem Kolben 5 in die Ausgangsposition selbsttätig zurückgeführt, wobei in der Pumpkammer 6 ein Unterdruck entsteht, so dass durch die durch das Ventil 9 verschlossene Öffnung 10 aus dem Vorratsbehältnis 2 Flüssigkeit in die Pumpkammer 6 nachströmt. Das Ventil 36 kann aus bakterizid wirkenden Materialien oder mit einem bakterizid wirkenden Stoff beschichteten Materialien gebildet sein (z.B. Silber oder Silbersalze), ebenso das Einlassventil 9, so dass eine effiziente Sterilhaltung des beinhalteten Flüssigkeitspräparates gewährleistet ist. Bei der vorstehend beschriebenen Ausführungsform ist insbesondere darauf hinzuweisen, dass dadurch, dass das Auslassventil 36 so ausgebildet ist, dass die Feder 33 in der Aussparung 34 geführt ist, ein sicheres und steriles Betätigen des Auslassventils möglich ist, da die Flüssigkeit nicht mit der Feder 33 in Kontakt kommt.

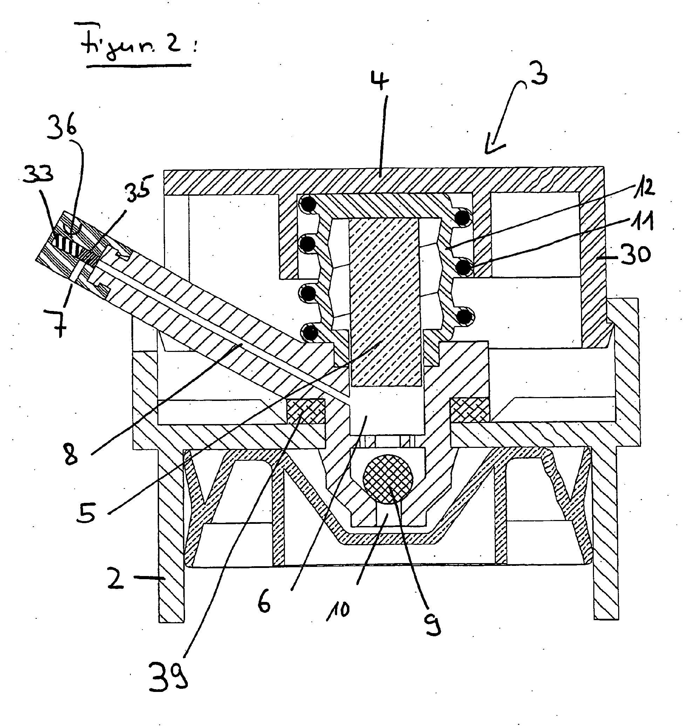

Figur 2 zeigt eine weitere optimierte Ausführungsform des Betätigungskörpers 3, der wiederum in vergrößerter Darstellung abgebildet ist. Im Unterschied zur Ausführungsform, wie sie in der Figur 1 beschrieben worden ist, wird bei dieser Ausführungsform nach der Figur 2 der Auslasskanal 8 geradlinig nach außen durch den zylindrischen Hohlkörper 30 geführt, was fertigungstechnisch eine Vereinfachung darstellt. Durch diese Ausführungsform ist ebenso gewährleistet, dass die Höhe der als Betätigungsknopf ausgebildeten Kappe 4 verringert werden kann, was den Betätigungskörper 3 und somit insgesamt die Dosiervorrichtung 1 kompakter werden lässt. Eine weitere Besonderheit des in der Figur 2 dargestellten Betätigungskörpers ist darin zu sehen, dass das Auslassventil 36, das wiederum eine Ventilfeder 33 und einen Ventilkörper 35 umfasst, sowie die Auslassdüse 7 in einem auf dem Auslasskanal 8 aufgeschnappten Gehäuse untergebracht ist. Durch diese leicht zu lösende mechanische Verbindung kann beispielsweise im Falle einer Verstopfung des Ventils eine leichte Zugänglichkeit gegeben sein, so dass das Auslassventil 36 ohne großen Aufwand gereinigt werden kann. An dieser Stelle ist auch noch darauf hinzuweisen, dass der Betätigungskörper 3 der erfindungsgemäßen Dosiervorrichtung, wie auch bereits die vorstehend bei Figur 1 beschriebene Ausführungsform ein zusätzliches Dichtelement 39 aufweist, das zur Abdichtung des Betätigungskörpers 3 gegenüber dem Vorratsbehältnis 2 dient.

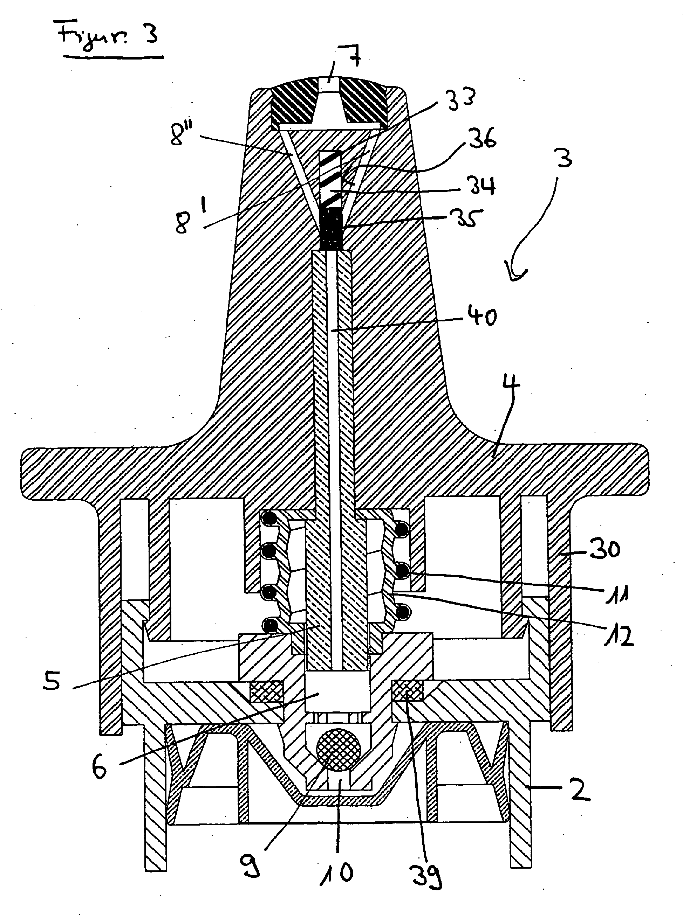

Figur 3 zeigt wiederum in vergrößerter Darstellung eine weitere Ausführungsform des Betätigungskörpers 3 der erfindungsgemäßen Dosiervorrichtung. Der Betätigungskörper 3 dieser Ausführungsform ist wiederum aus einer Kappe 4, die monolithisch mit einem zylindrischen Hohlkörper 3 verbunden ist, aufgebaut. Im Unterschied zu den Ausführungsformen nach den Figuren 1 und 2 ist nun hier die Düse 7 über die Kappe 4 nach außen geführt. Zur Realisierung ist es dabei nur erforderlich, dass in die Kappe 4 ein Steigrohr 40 eingearbeitet ist, wobei das Steigrohr 40 mit den beiden Teilauslasskanälen 8' und 8" über das Ventil 36 kommunizierend verbunden ist. Auch bei dieser Ausführungsform ist das Ventil 36 so aufgebaut, dass es aus einer Feder 33 und einem Ventilkolben 35 besteht. Selbstverständlich umfasst die Erfindung auch Ausführungsformen, bei der nur ein Auslasskanal 8' oder auch mehr wie zwei Auslasskanäle vorgesehen sind.

Die weiteren Bezugszeichen entsprechen denen, wie sie bereits bei den Figuren 1 und 2 beschrieben worden sind.

Figur 4 zeigt nun schematisch im Schnitt den Aufbau einer erfindungsgemäßen Dosiervorrichtung 1 aus einem Betätigungskörper 3 und einem Vorratsbehältnis 2. Der Aufbau des Betätigungskörpers 3 entspricht dabei dem, wie er bereits in Figur 1 im Detail beschrieben worden ist. Das Vorratsbehältnis 2 ist dabei so ausgebildet, dass es über einen Kolben 15 sowie eine Filtermatrix 16 verfügt. Die Filtermatrix 6 kann zusätzlich über eine Bodenverschraubung zugänglich sein.

Der Betätigungskörper 3 ist mit dem Vorratsbehältnis 2 über eine Steckverbindung verzahnt verbunden, der Verbund kann jedoch auch beliebig anderer Art, z.B. durch Verschraubung, erfolgen. In Figur 4a ist nun das Vorratsbehältnis 2 im entleerten Zustand dargestellt, d.h. der Schleppkolben 15 befindet sich direkt am Betätigungskörper 3, wohingegen die Figur 4b das Vorratsbehältnis 2 im gefüllten Zustand zeigt, d.h. der Schleppkolben 15 ist am Boden angeordnet. Bei der Ausführungsform nach Figur 4, die die einfachste Ausführung einer Schleppkolbenflasche zeigt, kann dabei auch vorgesehen sein, dass die Innenflächen des Vorratsbehältnisses 2 mit einer zusätzlichen reibungsvermindernden Beschichtung versehen ist. Die Beschichtung mit Polyethylen vermindert nicht nur die Reibung, sondern sie bietet auch den Vorteil, für medizinische Verwendungszwecke zugelassen zu sein.

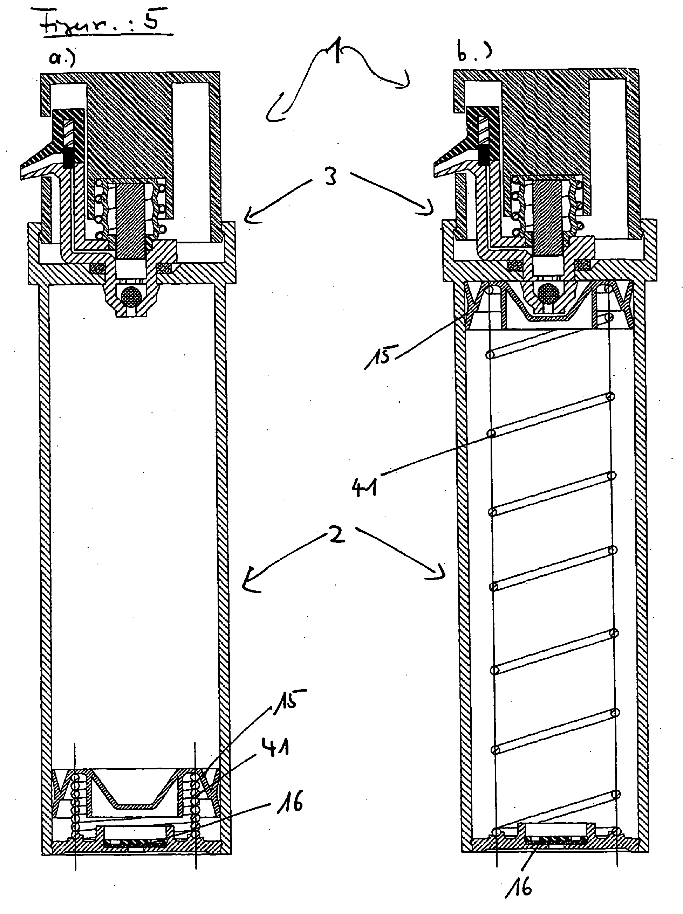

Figur 5 zeigt nun eine weitere Ausführungsform, die der entspricht, wie sie bereits bei Figur 4 beschrieben worden ist, jedoch ist hier zusätzlich vorgesehen, dass unter dem Schleppkolben 15 eine Druckfeder 41 angeordnet ist. Die Figur 5a zeigt dabei das Vorratsbehältnis 2 im gefüllten Zustand und die Figur 5b das Vorratsbehältnis 2 im entleerten Zustand, wobei hier dann die Druckfeder 41 den Schleppkolben 15 nach oben führt.

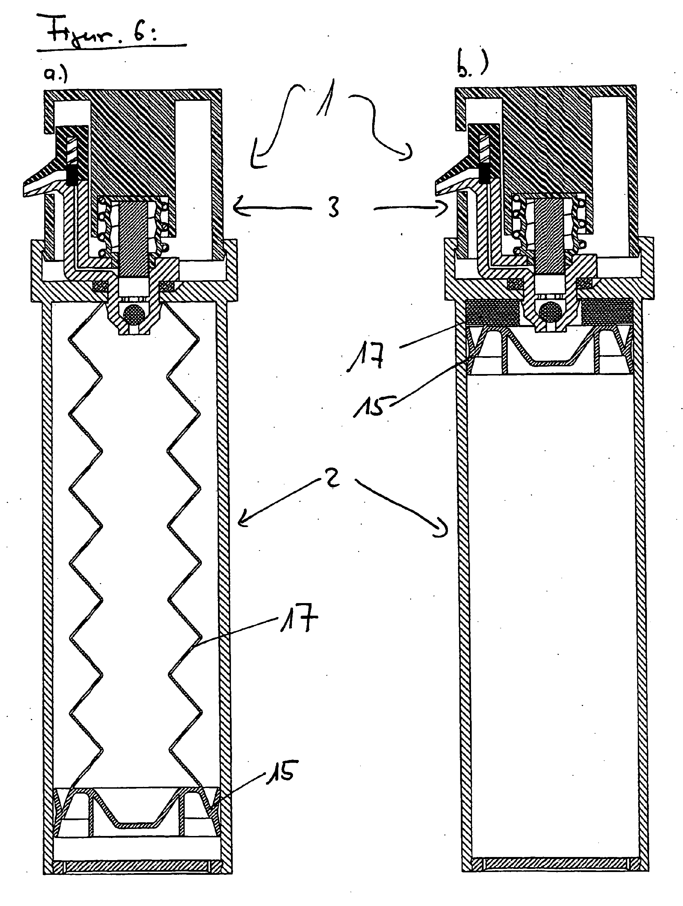

Figur 6 zeigt nun eine weitere Ausführungsform, wobei hier im Vorratsbehältnis 2 ein Faltenbalg 17 vorgesehen ist. Der Faltenbalg 17 hat dabei eine Vorspannung, d.h. im entleerten Zustand (Figur 6b) ist er zusammengefaltet. In Figur 6a ist dabei der Zustand dargestellt, wenn das Vorratsbehältnis 2 gefüllt ist. In diesem Falle ist dann der Faltenbalg 17 auseinandergefaltet. Bei der in Figur 6 dargestellten Ausführungsform ist nun im Unterschied zu der Ausführungsform nach Figur 5 keine zusätzliche Filtereinrichtung am Boden vorgesehen.

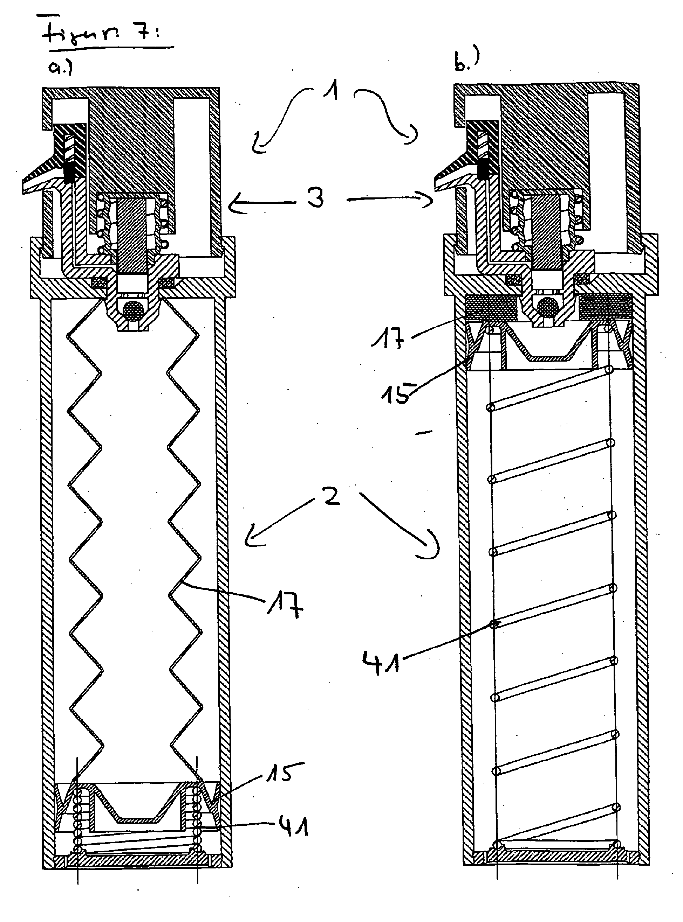

Figur 7 zeigt letztlich eine weitere Ausführungsform, die der entspricht, wie sie bereits in Figur 6 beschrieben worden ist, jedoch ist hier noch eine zusätzliche Druckfeder 41 unter dem Schleppkolben 15 vorgesehen. In dieser Ausführungsform ist somit neben dem Faltenbalg 17 eine zusätzliche Druckfeder 41 angeordnet.

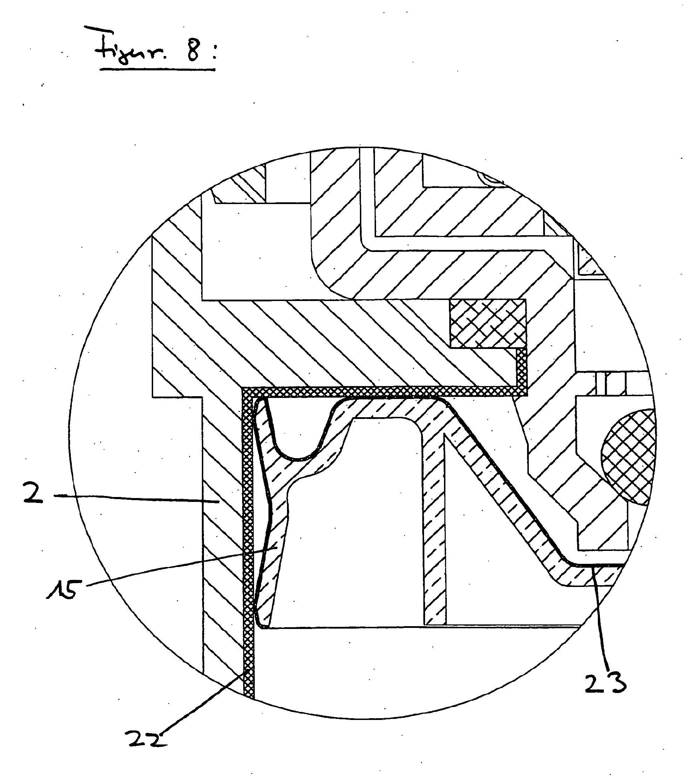

Figur 8 zeigt nun einen Ausschnitt aus dem Vorratsbehältnis nach der Figur 4, 5 bzw. 6 oder 7, wobei hier zusätzlich der Schleppkolben 15 auf seiner der Innenwand des Behälters 2 zugewandten Seite mit einer Gleitschicht, z.B. einer Polyethylenbeschichtung 23 ausgestattet ist. Weiterhin weist die Behälterinnenseite 2 des Vorratsbehältnisses eine zusätzliche Gleitschicht 22 auf. Die Beschichtung mit Polyethylen vermindert nicht nur die Reibung, sondern sie bietet auch den Vorteil, für medizinische Verwendungszwecke zugelassen zu sein.

## Patentansprüche

1. Dosiervorrichtung (1) zur dosierten Abgabe von Flüssigpräparaten, umfassend ein Vorratsbehältnis (2) für das Flüssigpräparat und einen mit dem Vorratsbehältnis (2) verbindbaren Betätigungskörper (3), wobei der Betätigungskörper (3) einen mit einer Kappe (4) verbundenen Hohlkörper (30), einen Pumpkolben (5), eine Pumpkammer (6) sowie eine mit der Pumpkammer (6) über einen Auslasskanal (8) in Verbindung stehende Düse (7) aufweist, wobei im Bereich des Endes des Auslasskanals (8) ein federbetriebenes Auslassventil (36) angeordnet ist, dessen Feder (33) in einer separaten, mit dem Auslasskanal (8) in Verbindung stehenden Aussparung (34) geführt ist, **dadurch gekennzeichnet dass** zwischen der Pumpkammer (6) und der Kappe (4) als mechanische Rückstellvorrichtung zur Rückstellung der Kappe (4) ein Faltenbalg (12) oder ein Faltenbalg (12) mit Rückstellfeder (11) angeordnet ist.

2. Dosiervorrichtung nach Anspruch 1, **dadurch gekennzeichnet, dass** die Düse (7) seitlich aus dem Hohlkörper (30) herausgeführt ist.

3. Dosiervorrichtung nach Anspruch 1, **dadurch gekennzeichnet, dass** die Düse (7) über die Kappe (4) herausgeführt ist.

4. Dosiervorrichtung nach einem der vorhergehenden Ansprüche, **dadurch gekennzeichnet, dass** die mit
dem Auslasskanal (8) in Verbindung stehende Düse (7) als separates Bauteil ausgebildet ist.

5. Dosiervorrichtung nach einem der vorhergehenden Ansprüche, **dadurch gekennzeichnet, dass** das Auslassventil (36) unmittelbar am Ende des Auslasskanals (8) angeordnet ist.

6. Dosiervorrichtung nach mindestens einem der Ansprüche 1 bis 5, **dadurch gekennzeichnet, dass** der Auslasskanal (8) und/oder der Ventilkörper (35) des Auslassventils (36) ein bakterizid wirkendes Mittel aufweist.

7. Dosiervorrichtung nach Anspruch 6, **dadurch gekennzeichnet, dass** das bakterizid wirkende Mittel ausgewählt ist aus der Gruppe bestehend aus bakterizid wirkenden Beschichtungen oder bakterizid wirkenden Einbauten.

8. Dosiervorrichtung nach Anspruch 6 oder 7, **dadurch gekennzeichnet, dass** das bakterizid wirkende Mittel Silber oder ein Silbersalz, insbesondere Silberchlorid ist.

9. Dosiervorrichtung nach einem der vorhergehenden Ansprüche, **dadurch gekennzeichnet, dass** der mit der Kappe (4) verbundene Hohlkörper (30) zylindrisch ist.

10. Dosiervorrichtung nach einem der vorhergehenden Ansprüche, **dadurch gekennzeichnet, dass** die Kappe (4) und der Hohlkörper (30) einstückig ausgebildet sind.

11. Dosiervorrichtung nach dem Anspruch 10, **dadurch gekennzeichnet, dass** die Kappe (4), der Hohlkörper (30) und der Pumpkolben (5) einstückig ausgebildet sind.

12. Dosierungsvorrichtung (1) nach einem der vorhergehenden Ansprüche, **dadurch gekennzeichnet, dass** die Pumpkammer (6) über eine mit einem Einlassventil (9) versehene Einlassöffnung (10) mit dem Vorratsbehältnis (2) verbunden ist.

13. Dosierungsvorrichtung (1) nach einem der vorhergehenden Ansprüche, **dadurch gekennzeichnet, dass** sie luftausgleichsfrei arbeitend ausgebildet ist.

14. Dosierungsvorrichtung (1) nach einem der vorhergehenden Ansprüche, **dadurch gekennzeichnet, dass** das Vorratsbehältnis (2) einen Kolben (15) und eine Filtermatrix (16) aufweist.

15. Dosierungsvorrichtung (1) nach einem der vorhergehenden Ansprüche, **dadurch gekennzeichnet, dass** das Vorratsbehältnis (2) innenseitig eine reibungsvermindernde Ausstattung (22) aufweist.

16. Dosierungsvorrichtung (1) nach Anspruch 11 oder 12, **dadurch gekennzeichnet, dass** der Kolben (15) auf seiner der Innenseite des Vorratsbehältnisses (2) zugewandten Seite eine reibungsvermindernde Ausstattung (23) aufweist.

## Claims

1. Metering device (1) for metered dispensing of liquid preparations, comprising a storage container (2) for the liquid preparation and an actuation body (3) which can be connected to the storage container (2), the actuation body (3) having a hollow body (30) connected to a cap (4), a pump piston (5), a pump chamber (6) and also a nozzle (7) connected to the pump chamber (6) via an outlet channel (8), **characterised in that** a spring-operated outlet valve (36) is disposed in the region of the end of the outlet channel (8), the spring (33) of which is guided in a separate recess (34) connected to the outlet channel (8), bellows (12) or bellows (12) with a return spring (11) being disposed between the pump chamber (6) and the cap (4) as mechanical restoring device for the return of the cap (4).

2. Metering device according to claim 1, **characterised in that** the nozzle (7) is guided out laterally from the hollow body (30).

3. Metering device according to claim 1, **characterised in that** the nozzle (7) is guided out via the cap (4).

4. Metering device according to one of the preceding claims, **characterised in that** the nozzle (7) connected to the outlet channel (8) is configured as a separate component.

5. Metering device according to one of the preceding claims, **characterised in that** the outlet valve (36) is disposed directly at the end of the outlet channel (8).

6. Metering device according to at least one of the claims 1 to 5, **characterised in that** the outlet channel (8) and/or the valve body (35) of the outlet valve (36) has an agent acting as a bactericide.

7. Metering device according to claim 6, **characterised in that** the agent acting as a bactericide is selected from the group comprising coatings acting as a bactericide or inserts acting as a bactericide.

8. Metering device according to claim 6 or 7, **characterised in that** the agent acting as a bactericide is silver or a silver salt, in particular silver chloride.

9. Metering device according to one of the preceding claims, **characterised in that** the hollow body (30) connected to the cap (4) is cylindrical.

10. Metering device according to one of the preceding claims, **characterised in that** the cap (4) and the hollow body (30) are configured in one piece.

11. Metering device according to claim 10, **characterised in that** the cap (4), the hollow body (30) and the pump piston (5) are configured in one piece.

12. Metering device (1) according to one of the preceding claims, **characterised in that** the pump chamber (6) is connected via an inlet opening (10) provided with an inlet valve (9) to the storage container (2).

13. Metering device (1) according to one of the preceding claims, **characterised in that** it is configured to operate free of air equalisation.

14. Metering device (1) according to one of the preceding claims, **characterised in that** the storage container (2) has a piston (15) and a filter matrix (16).

15. Metering device (1) according to one of the preceding claims, **characterised in that** the storage container (2) has a friction-reducing finish (22) on the inside.

16. Metering device (1) according to claim 11 or 12, **characterised in that** the piston (15), on its side orientated towards the inside of the storage container (2), has a friction-reducing finish (23).

## Revendications

1. Dispositif de dosage (1) destiné à distribuer de manière dosée des préparations liquides, comportant un réservoir de stockage (2) pour la préparation liquide et un corps de manoeuvre (3) apte à être relié au réservoir de stockage (2), ledit corps de manoeuvre (3) comportant un corps creux (30) relié à une calotte (4), un piston de pompage (5), une chambre de pompage (6), ainsi qu'une buse (7) communiquant avec la chambre de pompage (6) via un conduit d'évacuation (8), une soupape d'évacuation (36), actionnée par un ressort (33) logé dans un évidement (34) séparé communiquant avec le conduit d'évacuation (8), étant disposée dans la zone de l'extrémité du conduit d'évacuation (8), **caractérisé en ce qu'**un soufflet (12) ou un soufflet (12) avec ressort de rappel (11) est agencé entre la chambre de pompage (6) et la calotte (4) pour former un dispositif de rappel mécanique destiné à ramener la calotte (4) dans sa position initiale.

2. Dispositif de dosage selon la revendication 1, **caractérisé en ce que** la buse (7) s'engage latéralement hors du corps creux (30).

3. Dispositif de dosage selon la revendication 1, **caractérisé en ce que** la buse (7) s'engage vers l'extérieur via la calotte (4).

4. Dispositif de dosage selon l'une quelconque des revendications précédentes, **caractérisé en ce que** la buse (7), communiquant avec le conduit d'évacuation (8), est réalisée sous la forme d'un élément séparé.

5. Dispositif de dosage selon l'une quelconque des revendications précédentes, **caractérisé en ce que** la soupape d'évacuation (36) est disposée directement au niveau de l'extrémité du conduit d'évacuation (8).

6. Dispositif de dosage selon l'une quelconque des revendications précédentes 1 à 5, **caractérisé en ce que** le conduit d'évacuation (8) et/ou le corps de soupape (35) de la soupape d'évacuation (36) comportent un agent à action bactéricide.

7. Dispositif de dosage selon la revendication 6, **caractérisé en ce que** l'agent à action bactéricide est choisi dans le groupe formé par des revêtements à action bactéricide ou des éléments incorporés à action bactéricide.

8. Dispositif de dosage selon la revendication 6 ou 7, **caractérisé en ce que** l'agent à action bactéricide est l'argent ou un sel d'argent, en particulier un chlorure d'argent.

9. Dispositif de dosage selon l'une quelconque des revendications précédentes, **caractérisé en ce que** le corps creux (30) relié à la calotte (4) est cylindrique.

10. Dispositif de dosage selon l'une quelconque des revendications précédentes, **caractérisé en ce que** la calotte (4) et le corps creux (30) sont réalisés d'un seul tenant.

11. Dispositif de dosage selon la revendication 10, **caractérisé en ce que** la calotte (4), le corps creux (30) et le piston de pompage (5) sont réalisés d'un seul tenant.

12. Dispositif de dosage (1) selon l'une quelconque des revendications précédentes, **caractérisé en ce que** la chambre de pompage (6) est reliée au réservoir de stockage (2) par l'intermédiaire d'un orifice d'admission (10) muni d'une soupape d'admission (9).

13. Dispositif de dosage (1) selon l'une quelconque des revendications précédentes, **caractérisé en ce que** ledit dispositif est configuré pour travailler sans compensation d'air.

14. Dispositif de dosage (1) selon l'une quelconque des revendications précédentes, **caractérisé en ce que** le réservoir de stockage (2) comporte un piston (15) et une matrice de filtrage (16).

15. Dispositif de dosage (1) selon l'une quelconque des revendications précédentes, **caractérisé en ce que** le réservoir de stockage (2) comporte sur la face intérieure une garniture (22) réduisant le frottement.

16. Dispositif de dosage (1) selon la revendication 11 ou 12, **caractérisé en ce que** le piston (15), sur sa face orientée vers la face intérieure du réservoir de stockage (2), comporte une garniture (23) réduisant le frottement.
